# FASCICULE DE BREVET EUROPEEN

(11) **EP 1 114 041 B1**
(45) Date de publication et mention de la délivrance du brevet: **31.03.2004**
(21) Numéro de dépôt: 99942978.0
(22) Date de dépôt: 15.09.1999
(51) Int. Cl.: C07D 311/72

(54) **PROCEDE DE PREPARATION DE L'ACETATE D'ALPHA-TOCOPHEROL**
VERFAHREN ZUR HERSTELLUNG DES ALPHA-TOCOPHEROL ACETATS
PROCESS FOR THE PREPARATION OF ALPHA-TOCOPHEROL ACETATE

(30) Priorité: 18.09.1998 FR 9811679
(43) Date de publication de la demande: 11.07.2001
(73) Titulaire: AVENTIS ANIMAL NUTRITION S.A., 92160 Antony (FR)
(72) Inventeur: DHAINAUT, Jildaz, F-69003 Lyon (FR); DURAND, Thierry, F-73190 Challes les Eaux (FR)
(74) Mandataire: Guerre, Dominique
(86) Numéro de dépôt international: PCT/FR1999/002196
(87) Numéro de publication internationale: WO 2000/017185

(56) Documents cités:
- EP-A- 0 087 576
- EP-A- 0 694 541
- EP-A- 0 782 993
- EP-A- 0 850 937
- EP-A- 0 924 208
- WO-A-98/21197
- FR-A- 2 126 214
- PATENT ABSTRACTS OF JAPAN vol. 009, no. 052 (C-269), 6 mars 1985 (1985-03-06) & JP 59 190987 A (MITSUI TOATSU KAGAKU K.K.)
- DATABASE WPI Week 8519 Derwent Publications Ltd., London, GB; AN 85-113110 XP002102854 & JP 60 054380 A (SUMITOMO CHEM. CO., LTD.)
- CHEMICAL ABSTRACTS, vol. 86, no. 13, 28 mars 1977 (1977-03-28) Columbus, Ohio, US; abstract no. 90092, XP002121807 & JP 51 080859 A (TEIJIN, LTD.)
- CHEMICAL ABSTRACTS, vol. 102, no. 17, 29 avril 1985 (1985-04-29) Columbus, Ohio, US; abstract no. 149553, XP002121808 & CS 205 951 A (CZECH.)
- DATABASE WPI Week 8745 Derwent Publications Ltd., London, GB; AN 87-318204 XP002102855 & JP 62 226976 A (EISAI CO., LTD.)

## Description

La présente invention concerne un nouveau procédé de préparation de l'acétate d'α-tocophérol.

Ce procédé comprend notamment une étape de condensation de la triméthylhydroquinone et de l'isophytol.

Il est connu par exemple selon les brevets japonais No 60064977, No 53144574, No 53015381 de condenser l'isophytol avec la triméthylhydroquinone en présence d'un acide de Lewis limité au chlorure de zinc, en présence d'un acide minéral choisi parmi les acides halogénés et l'acide polyphosphorique dans un solvant constitué par du chorure de méthylène et de l'acide acétique.

Il est également connu d'après les brevets japonais No 59190987 et No 48072168 de condenser la triméthylhydroquinone avec l'isophytol en présence d'un catalyseur à base de chlorure de zinc et d'un acide choisi parmi l'acide chlorhydrique ou l'acide trichloracétique; la réaction étant réalisée dans un solvant constitué d'un ester d'acétyle et notamment d'acétate d'isopropyle.

Il est enfin connu d'après le brevet japonais No 48072167 de condenser l'isophytol avec la triméthylhydroquinone dans les mêmes conditions que précédemment mais en éliminant l'eau de la réaction au cours de sa formation lors de la condensation. Le brevet japonais No 6226976 qui réalise la réduction de la triméthylbenzoquinone et la condensation avec l'isophytol dans le même solvant que précédemment c'est à dire l'acétate d'isopropyle élimine entre les deux étapes la présence d'eau de façon à éviter la présence de cette dernière lors de la condensation finale de triméthylhydroquinone avec l'isophytol.

La réaction peut être schématisée de la façon suivante : 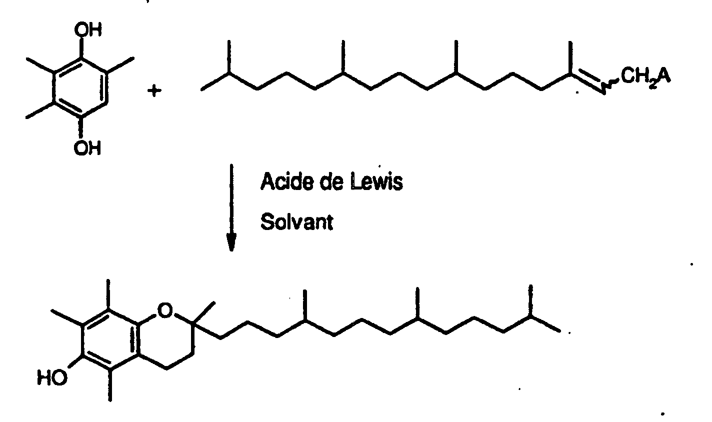 schéma dans lequel A représente un atome d'halogène ,un groupe hydroxyle, ou un groupe acétoxy.

Il est apparu de façon tout à fait surprenante que, si l'on voulait limiter la consommation en TMHQ à 1 éq molaire par rapport à l'isophytol, la présence d'eau dans l'étape de condensation avait un effet favorable sur la réaction contrairement à ce que laissait prévoir tout cet art antérieur. L'eau évite les réactions secondaires de la TMHQ en début de coulée d'isophytol.

Il est encore connu selon le brevet EP 0 850 937 qu'il est possible de condenser la TMHQ avec l'isophytol en présence d'eau à condition d'utiliser un solvant apolaire tel qu'un alcane. La quantité d'eau maximale utilisable sans nuire au rendement de la réaction et sans distiller l'eau formée en cours de réaction est limitée à 1.5 moles d'eau par mole d'isophytol. Dans ces conditions, t'influence de la quantité de chlorure de zinc utilisé n'a pas été étudiée.

Il est apparu que lorsque la mise en oeuvre du procédé préalablement décrit c'est à dire lorsque la catalyse avec le chlorure de zinc était réalisée en absence d'eau et dans un solvant polaire tel que les esters il y avait une perte importante de TMHQ par transestérification avec le solvant. Une partie du procédé de la présente invention permet de vaincre ce problème et permet de réaliser la condensation de la TMHQ avec l'isophytol dans un solvant polaire du type ester et en présence d'eau.

Une partie du procédé de la présente invention consiste donc à réaliser la condensation d'un phytol avec la triméthylhydroquinone dans un solvant polaire du type ester et en présence d'un acide de Brônsted et d'un halogénure de zinc caractérisé en ce que la réaction est réalisée en présence d'une quantité d'eau comprise entre 0.7 éq molaire et 2 éq molaire par rapport au nombre de moles d'halogénure de zinc et en présence d'une quantité d'halogénure de zinc supérieure à 0.3 équivalent molaire par rapport au phytol.

La présence de cette quantité d'eau a de nombreux avantages :
- elle permet d'augmenter le rendement de la réaction d'environ 4%,
- elle permet le recyclage de l'halogénure de zinc,
- à stoechiométrie équivalente en TMHQ et phytol, la présence d'eau augmente le rendement
- elle évite l'estérification de la TMHQ par le solvant.

De plus, dans le cas des solvants de la famille des esters, contrairement au cas des hydrocarbures décrits dans le brevet EP 0 850 937, la présence d'eau impose l'utilisation de quantités d'halogénures de zinc supérieure à 0.3 équivalent molaire par rapport au phytol, de manière à :
- Conserver un rendement > 92%
- Accélérer la cinétique de la réaction

Le phytol est choisi parmi l'isophytol ou un halogénure de phytyle tel que le bromure de phytyle, le chlorure de phytyle ou encore l'acétate de phytyle.

La réaction est notamment réalisée en présence d'un acide de Bronsted choisi parmi l'acide chlorhydrique ou sulfurique. On préfère utiliser l'acide chlorhydrique.

La réaction est réalisée en présence d'un solvant polaire permettant de solubiliser la triméthylhydroquinone et le phytol utilisés. Parmi les solvants polaires du type ester utilisables on peut citer l'acétate d'éthyle, de propyle, d'isopropyle, de butyle, d'isobutyle, les acétates ayant une chaîne plus longue ne sont pas préférés bien qu'ils soient utilisables, simplement la viscosité du solvant augmente avec la longueur de la chaîne ce qui n'est pas très favorable à la réaction. Les esters d'acides organiques plus longs que les acétates sont également utilisables, notamment les esters de l'acide propionique, butyrique ou isobutyrique, valérique ou isovalérique, mais comme dans le cas des acétates, l'augmentation de la longueur de la chaîne augmente la viscosité du milieu ce qui n'est pas toujours favorable à la réaction. Parmi tous ces esters on préfère utiliser l'acétate d'isopropyle.

La présence d'eau dans le milieu de réaction qui améliore le rendement de condensation entraîne dans certains cas la présence d'un système biphasique. Dans ce cas il est avantageux d'ajouter pour éviter la séparation des phases, un acide organique choisi parmi l'acide acétique, l'acide propionique ou l'acide butyrique. On préfère utiliser l'acide correspondant à l'ester utilisé comme solvant. Ainsi lorsqu'on utilise un acétate on préfère ajouter de l'acide acétique.

La quantité d'acide organique ajouté correspond à environ 3 à 20 fois la quantité pondérale d'eau présente dans le milieu.

Le catalyseur utilisé pour favoriser la condensation est choisi parmi les halogénures de zinc. On préfère utiliser le chlorure de zinc. II est avantageux d'utiliser aussi un acide de Bronsted choisi parmi l'acide chlorhydrique ou sulfurique.

Le catalyseur est utilisé selon une meilleure manière de mettre en oeuvre l'invention selon un rapport d'environ 0.7 à 1.2 équivalent par mole de phytol.

L'avantage d'utiliser cette quantité d'acide de Lewis par rapport à l'art antérieur qui en utilise moins est :
- d'augmenter la vitesse de réaction,
- d'améliorer la sélectivité de la condensation.

Le rapport molaire entre l'hydroquinone et le phytol est de préférence compris entre 1 et 1.5, il est tout préférentiellement compris entre 1 et 1.2. L'acide de Bronsted est utilisé de préférence selon une quantité molaire comprise entre 4 % et 16 % par rapport au nombre de moles de phytol.

Pour une meilleure mise en oeuvre de l'invention on préfère travailler à une température comprise entre 55°C et 75°C.

L'∝-tocophérol obtenu est séparé du milieu réactionnel par extraction liquide/liquide.

L'étape suivante du procédé de préparation de la vitamine E lorsqu'elle se présente sous forme d'acétate consiste à réaliser l'acétylation de l'∝-tocophérol.

Cette étape est réalisée selon une nouvelle méthode qui consiste à acétyler l'∝-tocophérol avec l'anhydride acétique en l'absence de tout solvant c'est à dire en masse.

L'acétylation est réalisée en présence d'un catalyseur constitué d'un acide inorganique choisi parmi l'acide sulfurique, l'acide phosphorique ou d'un acétate alcalin.

La présente invention concerne donc en premier lieu un procédé de préparation de l'acétate d'α-tocophérol, caractérisé en ce qu'on met en contact l'α-tocophérol avec de l'anhydride acétique, en présence d'un catalyseur d'acétylation choisi parmi l'acide sulfurique, l'acide phosphorique et les acétates alcalins, et en l'absence de tout autre solvant.

Il a été découvert qu'il était préférable d'employer l'acide phosphorique ou l'acétate de sodium car ces catalyseurs permettent d'éviter complètement la coloration du milieu réactionnel au cours de l'acétylation. L'acétate de tocophéryle obtenu est même plus clair que le tocophérol de départ. Il est préférable d'utiliser un rapport molaire entre l'anhydride acétique et le tocophérol compris entre 1 et 1.8. On utilise de préférence 0.7 à 2 % molaire d'acide comme catalyseur d'acétylation lorsque celui ci est l'acide sulfurique, 1 à 2 % molaire lorsque le catalyseur d'acétylation est l'acide phosphorique et 5 à 10 % molaire lorsque le catalyseur d'acétylation est l'acétate de sodium.

La présente invention concerne également un procédé complet de préparation de la vitamine E sous forme d'acétate au départ de triméthylbenzoquinone, l'ensemble du procédé étant réalisé dans le même solvant qui est un bon solvant de la triméthylbenzoquinone, de la triméthylhydroquinone et du phytol. Ce solvant est un solvant polaire du type ester et tout préférentiellement l'acétate d'isopropyle.

Elle consiste dans une première étape à réaliser une hydrogénation de la triméthylbenzoquinone avec un catalyseur d'hydrogénation, de préférence supporté, choisi parmi le palladium et le platine. On préfère utiliser le palladium supporté sur charbon.

La deuxième étape consiste, après filtration, de façon à éliminer le catalyseur, à réaliser la condensation de la triméthylhydroquinone obtenue à la première étape avec un phytol dans les conditions décrites précédemment et notamment en présence d'un halogénure de zinc, d'un acide de Bronsted et d'eau et tout particulièrement en présence de chlorure de zinc, d'acide chlorhydrique et d'eau. En fin de réaction le catalyseur est extrait par de l'eau, cette phase aqueuse est concentrée notamment de 80 % p à 91 % p ZnCl2, de manière à ne laisser qu'au maximum, en comptant l'eau venant de l'acide de Bronsted, deux moles d'eau par mole d'halogénure de zinc, et est recyclée à la deuxième étape du procédé.

La phase organique est alors concentrée à sec de façon à éliminer le solvant de réaction et ses sous produits éventuels puis acétylée en masse comme décrit ci-avant.

Le procédé d'extraction de l'acétate de vitamine E est ensuite réalisé de façon classique et connue de l'homme de l'art. Le milieu est extrait par un solvant peu ou pas miscible à l'eau, puis lavé avec une solution acide de façon à hydrolyser l'anhydride acétique subsistant, puis lavé en milieu alcalin pour désacétyler les acétates de la TMHQ. Par une séparation biphasique on isole la phase aqueuse contenant le sel alcalin de la TMHQ, on acidifie cette phase et on extrait la TMHQ avec le solvant de réaction, c'est à dire l'ester qui permet son recyclage à l'étape de condensation.

L'invention sera plus complètement décrite à l'aide des exemples suivants qui ne doivent pas être considérés comme limitatifs de l'invention.

### EXEMPLES

### Préparation de TMHQ en solution par hydrogénation

### Exemple 1 :

Dans un réacteur d'hydrogénation de 8 L, préalablement inerté à l'azote est chargé 3.1 g de Pd/C (3 %, 52 % H2O) et 3550 g d'ACIP. Le réacteur est mis sous 0.5 bar d'hydrogène sous agitation. Le milieu est chauffé à 80°C et maintenu 10 minutes à cette température.

Le réacteur est ensuite pressurisé jusqu'à 2 bars d'hydrogène. L'hydrogénation est réalisée en semi continu par addition simultanée de TMBQ à 96.5 % p/p (charge totale=700.3 g) et d'hydrogène de maniére à rester à 80°C et sous 2 bars d'hydrogène. La fin de réaction est détectée par la chute du débit d'hydrogène.

Le milieu réactionnel est dégazé puis purgé à l'azote. Le milieu réactionnel est filtré à chaud sous pression d'azote. On obtient une solution de 708.2 g de TMHQ dans l'ACIP prête à l'emploi pour la réaction de condensation. Le rendement de l'hydrogénation est de 99.8 %.

### Réactions de condensation TMHQ/Isophytol en l'absence d'eau.

### Exemple 2:

Une solution chaude de 76 g de TMHQ dans l'ACIP à 16 % p/p ACIP est chargée sous agitation dans un réacteur de 1 L. Cette solution est concentrée sous agitation jusqu'à 38 % p/p par distillation de l'ACIP à 45°C sous vide. A la suspension obtenue sont rajoutés 50.1 g de ZnCl2 98 % p/p dissouts dans 72 g d'acide acétique à chaud puis 4 g d'acide sulfurique 92 % p/p. Le milieu réactionnel est chauffé à 75°C. 162 g d'isophytol 91.5 % p/p sont alors coulés en 43 mn. En fin de coulée, la réaction est maintenue à 75°C pendant 60 mn. 100 g d'eau sont chargés sous agitation, le mélange est ensuite décanté. Les produits volatils de la phase organique sont éliminés par distillation à 75°C sous vide. 1 g d'acide sulfurique 92 % p/p sont chargés à 75°C puis 88.5 g d'anhydride acétique 98 % p/p sont coulés à cette température en 10 mn. Le milieu réactionnel est chauffé en 20 mn jusqu'à 105°C puis refroidi rapidement à 35°C. 1200 g d'hexane sont chargés sous agitation suivis de 71 g d'eau. Les deux phases sont décantées. La phase organique est lavée à nouveau par 71 g d'eau. La phase organique est évaporée au rotavapor à 60°C sous vide pendant 2 h.

On obtient 254.8 g d'acétate de tocophéryle (Toco) brut à 84.2 % p/p (rendement=90.9 %).

Le mode opératoire de l'Exemple 3 est identique à celui de l'Exemple 2. Seul le temps de coulée change (84 mn). Les paramètres sont indiqués dans le Tableau 1.

**Tableau 1:**

| Influence de l'absence d'eau. | | | | | | | | | |
|---|---|---|---|---|---|---|---|---|---|
| Exemple | temps maintien (mn) | AcOH (g) | ZnCl2 (éq) * | Acide (éq) | T(°C) | H2O (g) | H2O total ** (mol/mol ZnCl2) | Titre Toco (%) | Rdt Toco (% pur/pur) |
| 2 | 60 | 72 | 0.72 | H2SO4 (0.08) | 75 | 0 | 0.05 | 84.2 | 90.9 |
| 3 | 60 | 72 | 0.72 | H2SO4 (0.08) | 75 | 0 | 0.05 | 81.9 | 87.2 |

| | | | | | | | | | |
|---|---|---|---|---|---|---|---|---|---|
| TMHQ/Isophytol=1 éq molaire * les équivalents sont en moles par rapport au nombre de moles d'isophytol | | | | | | | | | |
| ** H2O total= H2O + H2O contenue dans H2SO4 92 %. | | | | | | | | | |

### Réactions de condensation TMHQ/Isophytol en présence d'eau. Réactions avec Acide=HCl 36% sans rajout d'eau supplémentaire

### Exemple 4:

Une solution chaude de 76 g de TMHQ dans l'ACIP à 16 % p/p ACIP est chargée sous agitation dans un réacteur de 1 L. Cette solution est concentrée sous agitation jusqu'à 38 % p/p par distillation de l'ACIP à 45°C sous vide. A la suspension obtenue sont rajoutés 50.1 g de ZnCl2 98 % p/p dissouts dans 72 g d'acide acétique à chaud puis 8.1 g d'acide chlorhydrique 36 % p/p. Le milieu réactionnel est chauffé à 75°C. 161.6 g d'isophytol 91.6 % p/p sont alors coulés en 47 mn. En fin de coulée, la réaction est maintenue à 75°C pendant 60 mn. 100 g d'eau sont chargés sous agitation, le mélange est ensuite décanté. Les produits volatils de la phase organique sont éliminés par distillation à 75°C sous vide. 1.1 g d'acide sulfurique 92 % p/p sont chargés à 75°C puis 88.6 g d'anhydride acétique 98 % p/p sont coulés à cette température en 10 mn. Le milieu réactionnel est chauffé en 30 mn jusqu'à 105°C puis refroidi rapidement à 35°C. 120 g d'hexane sont chargés sous agitation suivis de 70 g d'eau. Les deux phases sont décantées. La phase organique est lavée à nouveau par 70 g d'eau. La phase organique est évaporée au rotavapor à 60°C sous vide pendant 2 h.

On obtient 258 g d'acétate de tocophéryle (Toco) brut à 86.4 % p/p (rendement=94.3 %).

Les modes opératoires des Exemples 5 à 6 sont identiques à celui de l'Exemple 4. Les paramètres modifiés dans les exemples sont spécifiés dans le Tableau 2.

**Tableau 2:**

| Influence de la présence d'eau. | | | | | | | | | |
|---|---|---|---|---|---|---|---|---|---|
| Exemple | temps maintien (mn) | AcOH (g) | ZnCl2 (éq) * | Acide (éq) | T (°C) | H2O ajoutée (g) | H2O total ** (mol/mol ZnCl2) | TitreToco (%) | Rdt Toco (% pur/pur) |
| 4 | 60 | 72 | 0.72 | HCl 36% (0.16) | 75 | 0 | 0.75 | 86.4 | 94.3 |
| 5 | 60 | 72 | 0.72 | HCl 36% (0.16) | 75 | 0 | 0.75 | 87.8 | 93.4 |
| 6 | 90 | 47 | 0.45 | HCl 36% (0.09) | 62 | 0 | 0.75 | 89.5 | 97 |
| TMHQ/Isophytol=1 éq molaire | | | | | | | | | |

| | | | | | | | | | |
|---|---|---|---|---|---|---|---|---|---|
| * les équivalents sont en moles par rapport au nombre de moles d'isophytol | | | | | | | | | |
| ** H2O total= H2O + H2O contenue dans HCl 36 %. | | | | | | | | | |

### Réactions de condensation TMHQ/Isophytol en présence d'eau.

### Réactions avec Acide=HCl 36% ou H2SO4 92% avec rajout d'eau Influence de la teneur en eau et de la stoechiométrie en ZnCl2

### Exemple 7:

Une solution chaude de 76 g de TMHQ dans l'ACIP à 16 % p/p ACIP est chargée sous agitation dans un réacteur de 1 L. Cette solution est concentrée sous agitation jusqu'à 38 % p/p par distillation de l'ACIP à 45°C sous vide. A la suspension obtenue sont rajoutés 50.1 g de ZnCl2 98 % p/p dissouts dans 72 g d'acide acétique à chaud, 4.28 g d'acide sulfurique 92 % p/p et 5 g d'eau. Le milieu réactionnel est chauffé à 75°C. 162 g d'isophytol 91.5 % p/p sont alors coulés en 40 mn. En fin de coulée, la réaction est maintenue à 75°C pendant 60 mn. 200 g d'eau sont chargés sous agitation, le mélange est ensuite décanté. Les produits volatils de la phase organique sont éliminés par distillation à 75°C sous vide. 1.1 g d'acide sulfurique 92 % p/p sont chargés à 75°C puis 88.5 g d'anhydride acétique 98 % p/p sont coulés à cette température en 10 mn. Le milieu réactionnel est chauffé en 30 mn jusqu'à 105°C puis refroidi rapidement à 35°C. 200 g d'hexane sont chargés sous agitation suivis de 50 g d'eau. Les deux phases sont décantées. La phase organique est lavée à nouveau par 30 g d'eau. La phase organique est évaporée au rotavapor à 60°C sous vide pendant 2 h.

On obtient 253.2 g d'acétate de tocophéryle (Toco) brut à 88.2 % p/p (rendement=94.6 %).

Les modes opératoires des Exemples 8 à 15 sont identiques à celui de l'Exemple 7. L'acide sulfurique est parfois remplacé par de l'acide chlorhydrique. Les paramètres modifiés dans les Exemples sont spécifiés dans le Tableau 3.

**Tableau 3:**

| Influence de la teneur en eau et de la stoechiométrie en ZnCl2 | | | | | | | | | |
|---|---|---|---|---|---|---|---|---|---|
| Exemple | temps maintien (mn) | AcOH (g) | ZnCl2 (éq) * | Acide (éq) | T(°C) | H2O (g) | H2O total ** (mol/mol ZnCl2) | TitreToco (%) | Rdt Toco (% pur/pur) |
| 7 | 60 | 72 | 0.72 | H2SO4 (0.08) | 75 | 5 | 0.75 | 88.2 | 94.6 |
| 8 | 60 | 0 | 0.72 | H2SO4 (0.04) | 75 | 11 | 1.65 | 86.6 | 93.8 |
| 9 | 120 | 18 | 0.18 | HCl 36% (0.08) | 75 | 0 | 1.5 | 83.2 | 90.1 |
| 10 | 40 | 36 | 0.36 | H2SO4 (0.04) | 75 | 2.5 | 0.75 | 93.4 *** | 95.5 |
| 11 | 60 | 66 | 0.63 | H2SO4 (0.063) | 62 | 4.3 | 0.75 | 89.6 | 98.4 |
| 12 | 60 | 47 | 0.45 | H2SO4 (0.045) | 62 | 3.07 | 0.75 | 89.4 | 98.3 |
| 13 | 80 | 40 | 0.72 | HCl 36% (0.15) | 62 | 2.8 | 1.12 | 90.6 | 97.5 |
| 14 | 80 | 0 | 0.45 | HCl 36% (0.09) | 62 | 3.07 | 1.5 | 88.3 | 95.4 |
| 15 | 90 | 45 | 0.72 | HCl 36% (0.09) | 62 | 9.6 | 1.88 | 87.8 | 95.8 |

| | | | | | | | | | |
|---|---|---|---|---|---|---|---|---|---|
| * les équivalents sont en moles par rapport au nombre de moles d'isophytol TMHQ/Isophytol=1 éq molaire | | | | | | | | | |
| ** H2O total= H2O + H2O contenue dans l'acide | | | | | | | | | |
| *** Après purification partielle | | | | | | | | | |

### Réactions de condensation TMHQ/Isophytol en présence d'eau.

### Influence de la stoechiométrie en TMHQ et ZnCl2

### Exemple 16:

Une solution chaude de 76 g de TMHQ dans l'ACIP à 16 % p/p ACIP est chargée sous agitation dans un réacteur de 1 L. Cette solution est concentrée sous agitation jusqu'à 38 % p/p par distillation de l'ACIP à 45°C sous vide. A la suspension obtenue sont rajoutés 49.8 g de ZnCl2 98 % p/p dissouts dans 45 g d'acide acétique à chaud, 4.7 g d'acide chlorhydrique 36% p/p puis 4.8 g d'eau. Le milieu réactionnel est chauffé à 72°C. 160.5 g d'isophytol 92.2 % p/p sont alors coulés en 20 mn. En fin de coulée, la réaction est maintenue à 72°C pendant 40 mn. 100 g d'eau sont chargés sous agitation, le mélange est ensuite décanté. Un deuxième lavage est réalisé avec 60 g d'eau. Les produits volatils de la phase organique sont éliminés par distillation à 75°C sous vide. 0.6 g d'acide sulfurique 92 % p/p sont chargés à 85°C puis 68g d'anhydride acétique 92 % p/p sont coulés à cette température en 6 mn. Le milieu réactionnel est maintenu à cette température pendant 60 mn puis refroidi rapidement à 35°C. 240 g d'hexane sont chargés sous agitation suivis de 220 g d'eau et de 9 g de H2SO4 92 % p/p. Les deux phases sont décantées. La phase organique est alors lavée par un mélange de 100 g d'eau et 12g de NaOH 50 %. La phase organique est évaporée au rotavapor à 60°C sous vide pendant 2 h.

Après évaporation et purification partielle , on obtient 232.7 g d'acétate de tocophéryle (Toco) à 94.8 % p/p (rendement=93.3 %).

La TMHQ résiduelle est récupérée par acidification de la phase aqueuse alcaline par H2SO4 92 % p/p puis extraction à l'ACIP. La solution de TMHQ récupérée dans l'ACIP peut être recyclée en condensation TMHQ/Isophytol.

Les modes opératoires des Exemples 17 à 19 sont identiques à celui de l'Exemple 16. Les paramètres modifiés dans les Exemples sont spécifiés dans le Tableau 4.

**Tableau 4:**

| Influence de la teneur en eau et de la stoechiométrie en ZnCl2 | | | | | | | | | | |
|---|---|---|---|---|---|---|---|---|---|---|
| Exemple | temps maintien (mn) | AcOH (g) | TMHQ (éq) | ZnCl2 (éq) * | Acide (éq) | T (°C) | H2O (g) | H2O total ** (mol/mol ZnCl2) | TitreToco (%) | Rdt Toco (% pur/pur) |
| 16 | 40 | 45 | 1.01 | 0.72 | HCl 36% (0.09) | 72 | 4.8 | 1.12 | 94.7 *** | 93.3 |
| 17 | 40 | 45 | 1.05 | 0.72 | HCl 36% (0.09) | 75 | 4.8 | 1.12 | 95 *** | 95.7 |
| 18 | 20 | 45 | 1.15 | 0.72 | HCl 36% (0.09) | 75 | 4.8 | 1.12 | 93.2 *** | 96.2 |
| 19 | 40 | 62 | 1.15 | 1 | HCl 36% (0.09) | 75 | 7.6 | 1.12 | 96.4 *** | 98 |

| | | | | | | | | | | |
|---|---|---|---|---|---|---|---|---|---|---|
| * les équivalents sont en moles par rapport au nombre de moles d'isophytol | | | | | | | | | | |
| ** H2O total= H2O + H2O contenue dans l'acide | | | | | | | | | | |
| *** Après purification partielle | | | | | | | | | | |

### Réactions de condensation TMHQ/Isophytol en présence d'eau.

### Recyclage de ZnCl2

### Exemple 20:

Une solution chaude de 76 g de TMHQ dans l'ACIP à 16 % p/p ACIP est chargée sous agitation dans un réacteur de 1 L. Cette solution est concentrée sous agitation jusqu'à 38 % p/p par distillation de l'ACIP à 45°C sous vide. A la suspension obtenue sont rajoutés 49.8 g de ZnCl2 98 % p/p, 45 g d'acide acétique, 4.6 g d'acide chlorhydrique 36 % p/p puis 4.8 g d'eau. Le milieu réactionnel est chauffé à 62°C. 161.6 g d'isophytol 91.6 % p/p sont alors coulés en 60 mn. En fin de coulée, la réaction est maintenue à 62°C pendant 50 mn. 100 g d'eau sont chargés sous agitation, le mélange est ensuite décanté. Un deuxième lavage est réalisé avec 60 g d'eau. On récupère en tout 269.7 g de phase aqueuse contenant plus de 99.5 % du ZnCl2 chargé. Les produits volatils de la phase organique sont éliminés par distillation à 75°C sous vide. 0.7 g d'acide sulfurique 92 % p/p sont chargés à 70°C puis 68g d'anhydride acétique 92 % p/p sont coulés à cette température en 10 mn. Le milieu réactionnel est maintenu à cette température pendant 90 mn puis refroidi rapidement à 35°C. 240 g d'hexane sont chargés sous agitation suivis de 220 g d'eau et de 9 g de H2SO4 92 % p/p. Les deux phases sont décantées. La phase organique est alors lavée par un mélange de 100 g d'eau et 12g de NaOH 50 %. La phase organique est évaporée au rotavapor à 60°C sous vide pendant 2 h. Après évaporation et purification partielle , on obtient 237.8 g d'acétate de tocophéryle (Toco) à 96 % p/p (rendement=97 %).

La phase aqueuse (269.7 g) contenant ZnCl2 et HCl est évaporée au rotavapor sous un vide de 5 à 10 Torrs à environ 115°C jusqu'à obtention d'une suspension (∼90 % p/p de ZnCl2). A cette suspension sont ajoutés 45.6 g d'AcOH pour obtenir une solution. Le titre exact en ZnCl2 est dosé par colorimétrie. Cette solution de ZnCl2 est utilisée dans la réaction suivante de condensation TMHQ/Isophytol où la charge d'eau supplémentaire n'est plus nécessaire. Les quantités de réactifs (TMHQ, HCl 36 % et Isophytol) sont calculées sur la base de la quantité de ZnCl2 recyclée de manière à maintenir un rapport molaire ZnCl2/Isophytol de 0.72 éq.

Les modes opératoires des Exemples 21 à 23 sont identiques à celui de l'Exemple 20.

**Tableau 5:**

| Recyclage de ZnCl2 | | | | | | | | | | |
|---|---|---|---|---|---|---|---|---|---|---|
| Exemple | Recyclage | temps maintien (mn) | AcOH (g) | TMHC (éq) | ZnCl2 (éq)* | Acide (éq) | T (°C) | H2O total ** (mol/mol ZnCl2) | Titre Toco (%) | Rdt Toco (% pur/pur) |
| 20 | 0 | 50 | 45 | 1.01 | 0.73 | HCl 36% (0.09) | 62 | 1.16 | 96 *** | 97 |
| 21 | 1 | 50 | 45.6 | 1.01 | 0.73 | HCl 36% (0.09) | 62 | 1.14 | 95.6 *** | 97.2 |
| 22 | 2 | 50 | 43.6 | 1.01 | 0.70 | HCl 36% (0.09) | 62 | 1.25 | 95.3 *** | 97.2 |
| 23 | 3 | 50 | 42.75 | 1.01 | 0.68 | HCl 36% (0.09) | 62 | 1.34 | 95.8 *** | 97.2 |

| | | | | | | | | | | |
|---|---|---|---|---|---|---|---|---|---|---|
| * les équivalents sont en moles par rapport au nombre de moles d'isophytol | | | | | | | | | | |
| ** H2O total= H2O + H2O contenue dans l'acide | | | | | | | | | | |
| *** Après purification partielle | | | | | | | | | | |

### Catalyseurs d'acétylation : Influence sur la coloration de l'acétate de tocophéryle (Toco)

### Exemples 24 à 26 :

Le mode opératoire et les charges de la condensation TMHQ/Isophytol sont identiques à ceux de l'exemple 16.

Seule l'étape d'acétylation est changée. Les paramètres sont indiqués dans le Tableau 6.

**Tableau 6:**

| Influence du catalyseur d'acétylation sur la coloration du Toco | | | | | | | | |
|---|---|---|---|---|---|---|---|---|
| Exemple | catalyseur | éq cata (% mol) | Ac2O (éq) | T (°C) | t coulée Ac2O (mn) | t maintien (mn) | Titre Toca (%) | Rdt Toco (% pur/pur) |
| 24 | H2SO4 92 % | 2 | 1.3 | 70 | 7 | 90 | 89.9 | 97.5 |
| 25 | H3PO4 85% | 2 | 1.3 | 95 | 7 | 90 | 89.14 | 96.6 |
| 26 | AcONa | 10 | 1.3 | 110 | 7 | 90 | 89.43 | 98.6 |

Les colorations du Toco en solution ont été mesurées par la méthode Gardner.

Les échantillons de Toco sont dilués dans du cyclohexane. La solution est introduite dans une cellule quartz de 10 mm. L'analyse est effectuée dans la gamme spectrale 400 - 700 nm.

Les coordonnée trichromatiques sont les suivantes :

| Catalyseur | x | y | Y |
|---|---|---|---|
| H2SO4 | 0,5452 | 0,4325 | 0,84 |
| H3PO4 | 0,4553 | 0,4596 | 40,67 |
| AcONa | 0,4424 | 0,4594 | 67,71 |

La représentation de ces points sur la courbe Gardner correspondante est la suivante :

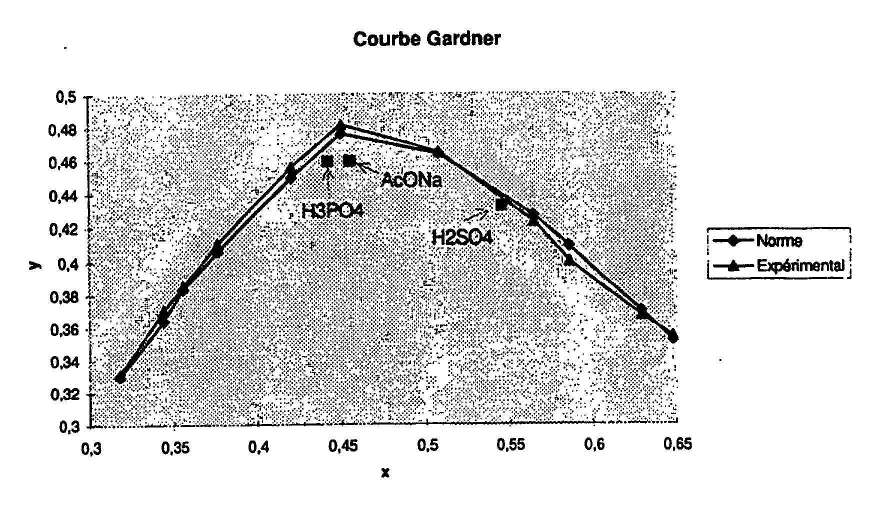

La courbe montre que H3PO4 et AcONa conduisent à des produits moins colorés que H2SO4.

## Revendications

1. Procédé de préparation de l'acétate d'α-tocophérol, **caractérisé en ce qu'**on met en contact l'α-tocophérol avec de l'anhydride acétique, en présence d'un catalyseur d'acétylation choisi parmi l'acide sulfurique, l'acide phosphorique et les acétates alcalins, et en l'absence de tout autre solvant.

2. Procédé selon la revendication 1, **caractérisé en ce que** le catalyseur d'acétylation est l'acétate de sodium.

3. Procédé selon la revendication 1 ou 2, **caractérisé en ce qu'**on utilise le catalyseur d'acétylation en une concentration molaire choisie parmi de 0,7% à 2% si c'est l'acide sulfurique, de 1% à 2% si c'est l'acide phosphorique et de 5% à 10% si c'est l'acétate de sodium.

4. Procédé selon l'une quelconque des revendications 1 à 3, **caractérisé en ce qu'**il comprend les étapes suivantes :
dans une première étape, on réalise l'hydrogénation de la triméthylbenzoquinone dans un solvant polaire de type ester, avec un catalyseur d'hydrogénation choisi parmi le palladium et le platine, pour obtenir la triméthylhydroquinone,
dans une seconde étape, on réalise, après filtration, la condensation de la triméthylhydroquinone avec un phytol, en présence d'un acide de Bronsted, d'un halogénure de zinc et d'eau, la quantité d'halogénure de zinc étant supérieure à 0,3 équivalent molaire par rapport au phytol et la quantité d'eau étant comprise entre 0,7 équivalent molaire et 2 équivalents molaire par rapport au nombre de moles d'halogénure de zinc,
dans une troisième étape, on extrait l'halogénure de zinc par de l'eau, et on concentre la solution aqueuse obtenue de manière à ne laisser subsister qu'au maximum deux moles d'eau par mole d'halogénure de zinc et on la recycle à la seconde étape du procédé,
dans une quatrième étape, on concentre la phase organique à sec et on acétyle en masse dans les conditions décrites à la revendication 1, et
on extrait, par des techniques connues, l'acétate d'α-tocophérol.

5. Procédé selon la revendication 4, **caractérisé en ce que** le solvant polaire de type ester est choisi parmi les acétates d'alkyle contenant de 2 à 4 atomes de carbone dans la chaîne alkyle.

6. Procédé selon la revendication 5, **caractérisé en ce que** le solvant polaire est l'acétate d'isopropyle.

7. Procédé selon la revendication 4, **caractérisé en ce que** le catalyseur d'hydrogénation est supporté.

8. Procédé selon la revendication 7, **caractérisé en ce que** le catalyseur est le palladium supporté sur charbon.

9. Procédé selon la revendication 4, **caractérisé en ce que** l'acide de Bronsted est choisi parmi l'acide chlorhydrique et l'acide sulfurique.

10. Procédé selon la revendication 9, **caractérisé en ce que** la quantité molaire de l'acide de Bronsted est comprise entre 4% et 16% par rapport au nombre de moles de phytol.

11. Procédé selon la revendication 4, **caractérisé en ce que** la quantité d'halogénure de zinc est comprise entre 0,7 et 1,2 équivalent molaire par rapport au phytol.

12. Procédé selon la revendication 4, **caractérisé en ce que** l'halogénure de zinc est le chlorure de zinc.

13. Procédé selon l'une quelconque des revendications 4 à 12, **caractérisé en ce que**, pour la seconde étape, on ajoute au milieu réactionnel un acide organique.

14. Procédé selon la revendication 13, **caractérisé en ce que** l'acide organique est l'acide acétique.

15. Procédé selon la revendication 13 ou 14,
**caractérisé en ce que** la quantité pondérale d'acide organique est comprise entre 3 à 20 fois la quantité pondérale d'eau présente dans le milieu.

16. Procédé selon l'une quelconque des revendications 4 à 15, **caractérisé en ce qu'**on extrait l'acétate d'α-tocophérol par un solvant peu ou non miscible à l'eau et on lave le milieu d'abord avec un acide pour hydrolyser l'anhydride acétique subsistant puis en milieu alcalin pour désacétyler les acétates de la triméthylhydroquinone.

17. Procédé selon la revendication 16, **caractérisé en ce qu'**on opère ensuite une séparation biphasique qui permet d'isoler la phase aqueuse contenant le sel alcalin de la triméthylhydroquinone, on acidifie cette phase, extrait la triméthylhydroqninone avec le solvant polaire de type ester et on recycle la solution organique obtenue à la seconde étape.

## Claims

1. Process for the preparation of α-tocopherol acetate, **characterized in that** the α-tocopherol is brought into contact with acetic anhydride in the presence of an acetylation catalyst selected from amongst sulphuric acid, phosphoric acid and the alkali metal acetates, in the absence of any other solvent.

2. Process according to Claim 1, **characterized in that** the acetylation catalyst is sodium acetate.

3. Process according to Claim 1 or 2, **characterized in that** the acetylation catalyst is used at a molar concentration of between 0.7% to 2% in the case of sulphuric acid, 1% to 2% in the case of phosphoric acid and 5% to 10% in the case of sodium acetate.

4. Process according to any of Claims 1 to 3, **characterized in that** it comprises the following steps:
in a first step, trimethylbenzoquinone is hydrogenated in a polar solvent of the ester type with a hydrogenation catalyst selected from amongst palladium and platinum, to obtain trimethylhydroquinone,
in a second step, the mixture is filtered and the trimethylhydroquinone is then subjected to condensation with a phytol in the presence of a Bronsted-acid, a zinc halide and water, the amount of zinc halide being above 0.3 mol equivalent based on the phytol and the amount of water being from 0.7 mol equivalent to 2 mol equivalent based on the number of moles of zinc halide,
in a third step, the zinc halide is extracted with water and the aqueous solution obtained is concentrated in such a way that not more than 2 mol of water per mole of zinc halide remain, and it is recirculated into the second process step,
in a fourth step, the organic phase is evaporated to dryness and acetylated in bulk under the conditions described in Claim 1, and
the a-tocopherol acetate is extracted by known techniques.

5. Process according to Claim 4, **characterized in that** the polar solvent of the ester type is selected amongst the alkyl acetates with 2 to 4 carbon atoms in the alkyl chain.

6. Process according to Claim 5, **characterized in that** the polar solvent is isopropyl acetate.

7. Process according to Claim 4, **characterized in that** the hydrogenation catalyst is a supported catalyst.

8. Process according to Claim 7, **characterized in that** the catalyst is palladium-on-charcoal.

9. Process according to Claim 4, **characterized in that** the Bronsted acid is selected amongst hydrochloric acid and sulphuric acid.

10. Process according to Claim 9, **characterized in that** the molar amount of Bronsted acid amounts to between 4% and 16% based on the number of moles of phytol.

11. Process according to Claim 4, **characterized in that** the amount of zinc halide amounts to between 0.7 and 1.2 mol equivalent based on the phytol.

12. Process according to Claim 4, **characterized in that** the zinc halide is zinc chloride.

13. Process according to any of Claims 4 to 12, **characterized in that**, for the second step, an organic acid is added to the reaction medium.

14. Process according to Claim 13, **characterized in that** the organic acid is acetic acid.

15. Process according to Claim 13 or 14, **characterized in that** the amount by weight of organic acid amounts to between 3 to 20 times of the amount by weight of water which is present in the reaction medium.

16. Process according to any of Claims 4 to 15, **characterized in that** the α-tocopherol acetate is extracted with a solvent which is sparingly miscible or immiscible with water, and the reaction mixture is first washed with an acid in order to hydrolyse the remaining acetic anhydride and subsequently in an alkaline medium in order to deacetylate the trimethylhydroquinone acetates.

17. Process according to Claim 17, **characterized in that** a biphasic separation is subsequently carried out, which allows the aqueous phase, which contains the alkali metal salt of trimethylhydroquinone to be isolated, this phase is acidified, the trimethylhydroquinone is extracted with the polar solvent of the ester type, and the organic solution obtained is recirculated into the second step.

## Patentansprüche

1. Verfahren zur Herstellung von α-Tocopherolacetat, **dadurch gekennzeichnet, daß** man α-Tocopherol in Gegenwart eines Acetylierungskatalysators aus der Gruppe Schwefelsäure, Phosphorsäure und Alkalimetallacetate sowie in Abwesenheit von anderen Lösungsmitteln mit Essigsäureanhydrid in Kontakt bringt.

2. Verfahren nach Anspruch 1, **dadurch gekennzeichnet, daß** es sich bei dem Acetylierungskatalysator um Natriumacetat handelt.

3. Verfahren nach Anspruch 1 oder 2, **dadurch gekennzeichnet, daß** man den Acetylierungskatalysator, wenn es sich um Schwefelsäure handelt, in einer molaren Konzentration von 0,7% bis 2%, wenn es sich um Phosphorsäure handelt, in einer Konzentration von 1% bis 2%, und wenn es sich um Natriumacetat handelt, in einer Konzentration von 5% bis 10%, verwendet.

4. Verfahren nach einem der Ansprüche 1 bis 3, **dadurch gekennzeichnet, daß** es die folgenden Schritte umfaßt:
in einem ersten Schritt wird Trimethylbenzochinon in einem polaren Lösungsmittel vom Ester-Typ mit einem Hydrierungskatalysator aus der Gruppe Palladium und Platin zu Trimethylhydrochinon hydriert,
in einem zweiten Schritt wird nach dem Filtrieren das Trimethylhydrochinon in Gegenwart von einer Bronsted-Säure, einem Zinkhalogenid und von Wasser mit einem Phytol kondensiert, wobei die Zinkhalogenidmenge über 0,3 Moläquivalent in bezug auf das Phytol beträgt und die Wassermenge 0,7 Moläquivalent bis 2 Moläquivalent in bezug auf die Anzahl der Mol Zinkhalogenid beträgt,
in einem dritten Schritt wird das Zinkhalogenid mit Wasser extrahiert und die erhaltene wäßrige Lösung wird so stark konzentriert, daß nicht mehr als zwei Mol Wasser pro Mol Zinkhalogenid verbleiben, und in den zweiten zurückgeleitet,
in einem vierten Schritt wird die organische Phase zur Trockne eingeengt und in Masse unter den in Anspruch 1 beschriebenen Bedingungen acetyliert, und
das α-Tocopherolacetat wird nach bekannten Verfahren extrahiert.

5. Verfahren nach Anspruch 4, **dadurch gekennzeichnet, daß** das polare Lösungsmittel vom Ester-Typ aus der Gruppe der Alkylacetate mit 2 bis 4 Kohlenstoffatomen in der Alkylkette stammt.

6. Verfahren nach Anspruch 5, **dadurch gekennzeichnet, daß** es sich bei dem polaren Lösungsmittel um Isopropylacetat handelt.

7. Verfahren nach Anspruch 4, **dadurch gekennzeichnet, daß** es sich bei dem Hydrierungskatalysator um einen Trägerkatalysator handelt.

8. Verfahren nach Anspruch 7, **dadurch gekennzeichnet, daß** es sich bei dem Katalysator um Palladium-auf-Kohle handelt.

9. Verfahren nach Anspruch 4, **dadurch gekennzeichnet, daß** die Bronsted-Säure aus der Gruppe Chlorwasserstoffsäure und Schwefelsäure stammt.

10. Verfahren nach Anspruch 9, **dadurch gekennzeichnet, daß** die Molmenge an Bronsted-Säure 4% bis 16% in bezug auf die Anzahl Mol Phytol beträgt.

11. Verfahren nach Anspruch 4, **dadurch gekennzeichnet, daß** die Zinkhalogenidmenge 0,7 bis 1,2 Moläquivalent in bezug auf das Phytol beträgt.

12. Verfahren nach Anspruch 4, **dadurch gekennzeichnet, daß** es sich bei dem Zinkhalogenid um Zinkchlorid handelt.

13. Verfahren nach einem der Ansprüche 4 bis 12, **dadurch gekennzeichnet, daß** man für den zweiten Schritt den Ansatz mit einer organischen Säure versetzt.

14. Verfahren nach Anspruch 13, **dadurch gekennzeichnet, daß** es sich bei der organischen Säure um Essigsäure handelt.

15. Verfahren nach Anspruch 13 oder 14, **dadurch gekennzeichnet, daß** die Gewichtsmenge an organischer Säure das 3- bis 20fache der Gewichtsmenge des in dem Ansatz vorhandenen Wassers beträgt.

16. Verfahren nach einem der Ansprüche 4 bis 15, **dadurch gekennzeichnet, daß** man das α-Tocopherolacetat mit einem wenig oder nicht wassermischbaren Lösungsmittel extrahiert und den Ansatz zuerst mit einer Säure wäscht, um das verbleibende Essigsäureanhydrid zu hydrolysieren, und dann alkalisch wäscht, um die Trimethylhydrochinonacetate zu desacetylieren.

17. Verfahren nach Anspruch 16, **dadurch gekennzeichnet, daß** man anschließend eine Trennung der beiden Phasen durchführt, wodurch man die wäßrige Phase, die das Trimethylhydrochinon-Alkalimetallsalz enthält, isolieren kann, diese Phase ansäuert, das Trimethylhydrochinon mit dem polaren Lösungsmittel vom Ester-Typ extrahiert und die erhaltene organische Lösung in den zweiten Schritt zurückführt.
